# EUROPEAN PATENT APPLICATION

(11) **EP 1 298 430 A2**
(43) Date of publication of application: **02.04.2003**
(21) Application number: 02021541.4
(22) Date of filing: 26.09.2002
(51) Int. Cl.: G01N 33/00

(54) **Gas sensor with error compensation**

(30) Priority: 28.09.2001 JP 2001302824
(71) Applicant: Denso Corporation, Kariya-city, Aichi-pref., 448-0029 (JP)
(72) Inventor: Katafuchi, Toru, Kariya-city, Aichi-pref., 448-0029 (JP); Mizutani, Keigo, Nishio-city, Aichi-pref., 445-0012 (JP)
(74) Representative: Leson, Thomas Johannes Alois, Dipl.-Ing.

(57) **Abstract**

A gas sensor is provided which includes a sensor cell. The sensor cell is made up of a solid electrolyte member and a positive and a negative electrode installed on the solid electrolyte member. The sensor cell works to produce an electric current produced by a flow of oxygen ions from the negative to positive electrode which arises from dissociation of oxygen molecules on the negative electrode. An oxygen discharge chamber is provided on the positive electrode to discharge the oxygen molecules from outside the positive electrode, thereby eliminating an error of a sensor output caused by a stay of the oxygen molecules around the positive electrode.

## Description

### BACKGROUND OF THE INVENTION

### 1 Technical Field of the Invention

The present invention relates generally to a gas sensor for measuring the concentration of, for example, NOx contained in exhaust gasses of an automotive internal combustion engine, and more particularly to an improved structure of such a gas sensor designed to minimize an error of a sensor output.

### 2 Background Art

Gas sensors are known which are used for measuring NOx contained in exhaust gasses of automotive engine for burning control of the engine and/or monitoring the deterioration of a catalytic converter. This type of gas sensor typically includes a sensor cell working to measure the concentration of NOx. The sensor cell is made up of a solid electrolyte member and a positive and a negative electrode installed on opposed surfaces of the solid electrolyte member. Application of voltage across the positive and negative electrodes of the sensor cell will cause NOx to be dissociated or ionized by the negative electrode, thereby producing oxygen ions which flow to the positive electrode as an ion current indicating the concentration of NOx. The oxygen ions become oxygen molecules on the positive electrode of the sensor cell which are, in turn, discharged outside the gas sensor.

When the oxygen molecules are discharged outside the positive electrode of the sensor cell, it may cause an ambient atmosphere to be changed to an oxygen-rich side, thereby resulting in a rise in pressure around the positive electrode of the sensor cell. This will result in an error in output of the sensor cell, thus decreasing the accuracy of measurement of the concentration of NOx. This problem is common to gas sensors which have a sensor cell working to dissociate or ionize a gas to be measured on a negative electrode to produce an ion current as a function of the concentration of the gas.

### SUMMARY OF THE INVENTION

It is therefore a principal object of the present invention to avoid the disadvantages of the prior art.

It is another object of the present invention to provide a gas sensor which is designed to minimize an error in determining the concentration of a specified component of a measurement gas.

According to one aspect of the invention, there is provided a gas sensor which may be used with an automotive control system designed to control burning of an internal combustion engine. The gas sensor comprises: (a) a gas chamber into which a measurement gas is introduced from outside the gas sensor; (b) a sensor cell made up of a solid electrolyte member and a positive and a negative electrode installed on the solid electrolyte member, said sensor cell working to produce an output as a function of concentration of a specified component of the measurement gas; (c) a monitor cell made up of a solid electrolyte member and a positive and a negative electrode installed on the solid electrolyte member, said monitor cell working to produce an output as a function of concentration of oxygen within said gas chamber; (d) a pump cell made up of a solid electrolyte member and a pair of oxygen pumping electrodes installed on the solid electrolyte member, said pump cell working to pump oxygen out of and into said gas chamber, selectively; and (e) an oxygen discharge chamber to which a portion of the positive electrode of at least one of said sensor cell and said monitor cell is exposed. The oxygen discharge chamber is defined by a member which is so dense as to arrest penetration of oxygen molecules therethrough. The oxygen discharge chamber works to discharge oxygen molecules appearing on the positive electrode of the at least one of the sensor cell and the monitor cell to, for example, the air, thereby avoiding a stay of the oxygen molecules around the positive electrode which may cause an ambient atmosphere to change to an oxygen-rich side, thereby resulting in a undesirable rise in pressure around the positive electrode.

In the preferred mode of the invention, the solid electrolyte members of said monitor cell and said sensor cell have outer surfaces which face the measurement gas existing outside the gas sensor and on which the positive electrodes of said monitor cell and said sensor cell are installed. The member is installed on the outer surfaces of the solid electrolyte members to define said oxygen discharge chamber to which the oxygen molecules appearing on the portion of the positive electrode of the at least one of said sensor cell and said monitor cell are discharged.

If a volume of said oxygen discharge chamber is defined as *V*, and an area of the portion of the positive electrode of the at least one of said sensor cell and said monitor cell exposed inside the oxygen discharge chamber is defined as *Se*, then a relation of 0.01 ≦ *V*/*Se* is met.

The height of the oxygen discharge chamber is 0.01mm or more.

If total area of the positive electrode of the at least one of the sensor cell and the monitor cell is defined as *S*, then a relation of 0.25 ≦ *Se*/*S* is met.

If a total length of the positive electrode of the at least one of the sensor cell and the monitor cell extending in a lengthwise direction of the gas sensor is defined as *L*, and a length of the portion of the positive electrode of the at least one of the sensor cell and the monitor cell exposed inside the oxygen discharge chamber extending in the lengthwise direction of the gas sensor is defined as *Le,* then a relation of 0.25 ≦ *Le*/*L* is met.

If a total width of the positive electrode of the at least one of the sensor cell and the monitor cell extending perpendicular to a length of the gas sensor is defined as *W*, and a width of the portion of the positive electrode of the at least one of the sensor cell and the monitor cell exposed inside the oxygen discharge chamber extending perpendicular to the length of the gas sensor is defined as *We,* then a relation of 0.25 ≦ *We*/*W* is met.

According to another aspect of the invention, there is provided a gas sensor which comprises: (a) a sensor cell made up of a solid electrolyte member and a positive and a negative electrode installed on the solid electrolyte member, the sensor cell working to produce an output as a function of concentration of a specified component of a measurement gas; (b) a monitor cell made up of a solid electrolyte member and a positive and a negative electrode installed on the solid electrolyte member, the monitor cell working to produce an output as a function of concentration of oxygen contained in the measurement gas; and (c) an oxygen discharge chamber to which a portion of the positive electrode of at least one of the sensor cell and the monitor cell is exposed.

In the preferred mode of the invention, the solid electrolyte members of the monitor cell and the sensor cell have outer surfaces which face the measurement gas existing outside the gas sensor and on which the positive electrodes of the monitor cell and the sensor cell are installed. The oxygen discharge chamber is defined by a member which is installed on the outer surfaces of the solid electrolyte members and works to discharge oxygen molecules appearing on the portion of the positive electrode of the at least one of the sensor cell and the monitor cell.

### BRIEF DESCRIPTION OF THE DRAWINGS

The present invention will be understood more fully from the detailed description given hereinbelow and from the accompanying drawings of the preferred embodiments of the invention, which, however, should not be taken to limit the invention to the specific embodiments but are for the purpose of explanation and understanding only.

In the drawings:
Fig. 1 is a vertical sectional view which shows an internal structure of a gas sensor to the invention;
Fig. 2 is a sectional view taken along the line *M-M* in Fig. 1;
Fig. 3 is a partially perspective view which shows positive electrodes of a sensor cell and a monitor cell disposed inside an oxygen discharge chamber;
Fig. 4 is a partially vertical sectional view which shows dimensions of a positive electrode of a monitor cell disposed inside an oxygen discharge chamber;
Fig. 5 is a plan view of Fig. 4;
Fig. 6 is a graph which shows an error in output of a sensor cell in terms of the height of an oxygen discharge chamber;
Fig. 7 is a graph which shows an error in output of a sensor cell in terms of a ratio of a length *Le* of a portion of a positive electrode of a sensor cell exposed inside an oxygen discharge chamber to a total length *L* of the positive electrode;
Fig. 8 is a graph which shows an error in output of a sensor cell in terms of a ratio of a width *We* of a portion of a positive electrode of a sensor cell exposed inside an oxygen discharge chamber to a total width *W* of the positive electrode;
Fig. 9 is a graph which shows outputs of a sensor cell and a monitor cell in the absence of an oxygen discharge chamber;
Fig. 10 is a graph which shows outputs of a sensor cell and a monitor cell in the presence of an oxygen discharge chamber
Fig. 11 is a graph which shows the concentration of NOx as determined using outputs of a sensor cell and a monitor cell in the presence of an oxygen discharge chamber;
Fig. 12 is a graph which shows the concentration of NOx as determined using outputs of a sensor cell and a monitor cell in the absence of an oxygen discharge chamber;
Fig. 13 is a transverse sectional view which shows a modification of positive electrodes of a sensor cell and a monitor cell;
Fig. 14 is a modification of an oxygen discharge chamber within which a porous member is disposed; and
Figs. 15, 16, and 17 are plan views which shows modifications of positive electrodes of a sensor cell and a monitor cell.

### DESCRIPTION OF THE PREFERRED EMBODIMENTS

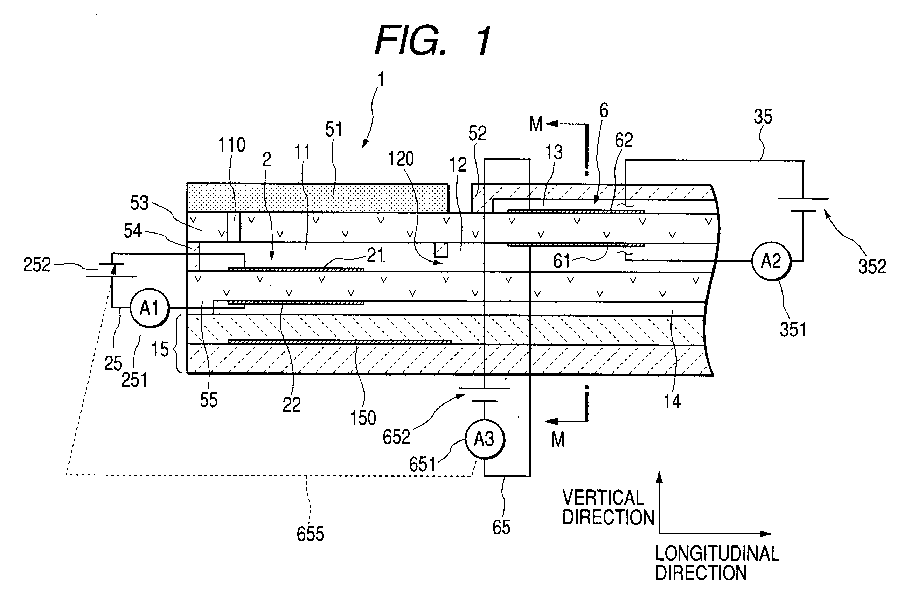

Referring now to the drawings, wherein like numbers refer to like parts in several views, particularly to Fig. 1, there is shown a gas sensor 1 according to the first embodiment of the invention which may be used with an automotive control system designed to control the quantity of fuel injected into an internal combustion gasoline engine as a function of an output of the gas sensor 1 under feedback (F/B) control to bring the air-fuel (A/F) ratio into agreement with a target one. The gas sensor 1 is installed in an exhaust pipe of an automotive vehicle and works to measure concentrations of oxygen (O₂) and nitrogen oxide (NOx) contained in exhaust gasses of the internal combustion engine simultaneously.

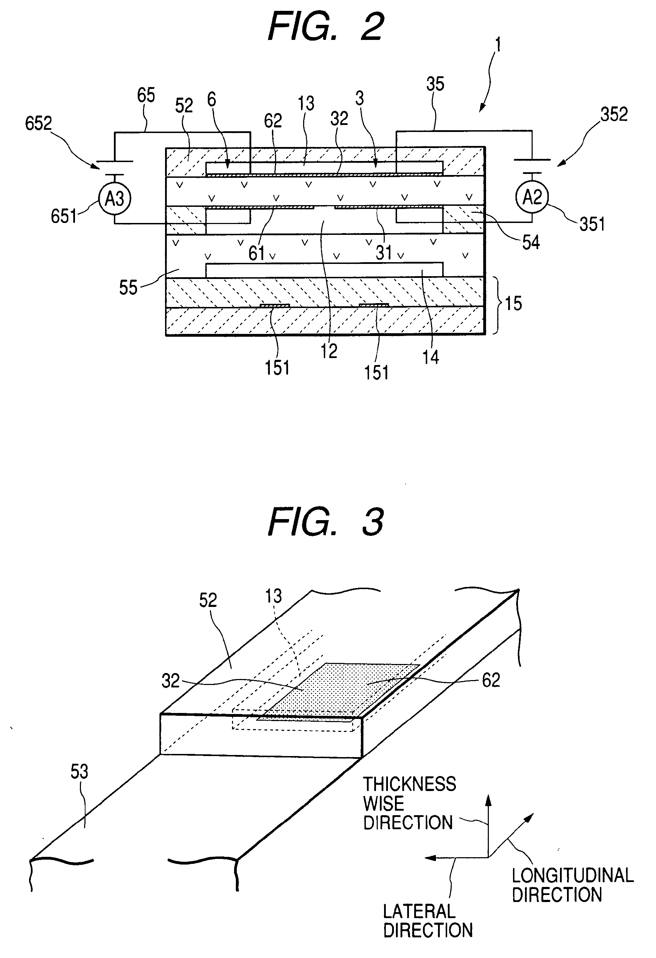

The gas sensor 1 has a monitor cell 6, as clearly shown in Fig. 2, which consists of a solid electrolyte layer 53, a negative terminal 61, and a positive electrode 62. The negative and positive terminals 61 and 62 are formed on opposite surfaces of the solid electrolyte layer 53. The surface of the positive terminal 62 is exposed to an oxygen discharge chamber 13. The monitor cell 6 works to measure the concentration of oxygen (O₂) contained in exhaust gasses of the engine (will also be referred to as a measurement gas below) entering a second gas chamber 12 through a first gas chamber 11.

The gas sensor 1, as shown in Figs. 1 and 2, also includes a pump cell 2 and a sensor cell 3. The sensor cell 3 is made up of a pair of electrodes 31 and 32 formed on the solid electrolyte layer 53. The sensor cell 3 works to measure the concentration of NOx contained in the measurement gas in the second gas chamber 12. The electrode 32 is, as clearly shown in Fig. 2, formed integrally with the electrode 62.

The pump cell 2 is made up of the solid electrolyte layer 53 and a pair of electrodes 21 and 22 formed on the solid electrolyte layer 53. The pump cell 2, as is well known in the art, works to dissociate or ionize and pump thereinto oxygen molecules (O₂) contained in the measurement gas entering the first gas chamber 11 and discharge them for measuring the concentration of oxygen (O₂) and also to dissociate or ionize and pump oxygen molecules within a reference gas chamber 14 into the first gas chamber 11 when the concentration of oxygen within the first gas chamber 11 is lower than a given level for keeping the concentration of oxygen within the first gas chamber 11 at the given level.

The oxygen discharge chamber 13 to which the positive electrode 32 of the sensor cell 3 and the positive electrode 62 of the monitor cell 6 are exposed is defined by a spacer 52 whose outer surface is exposed to the measurement gas existing outside the gas sensor 1. The spacer 52 is made of material which is so dense as to arrest the penetration of oxygen molecules therethrough.

The gas sensor 1 is made of a lamination of a heater 15, a second solid electrolyte layer 55, a spacer 54, the first solid electrolyte layer 53, a porous layer 51, and the spacer 52. The spacers 52 and 54 are made of alumina ceramic plates. The solid electrolyte layers 53 and 55 are made of an oxygen ion-conducting material such as zirconia ceramic. The heater 15 is made up of insulating alumina ceramic layers and a heating element 150 disposed therebetween. The heating element 150 has, as shown in Fig. 2, leads 151 for electric connection with a power supply (not shown).

The first solid electrolyte layer 53 has formed therein a pinhole defining a first diffusion resistance path 110 through which the measurement gas flowing around the gas sensor 1 is admitted into the first gas chamber 11. The porous layer 51 covers the first diffusion resistance path 110. The spacer 52 is disposed adjacent the porous layer 51. The spacer 52 and the first solid electrolyte layer 53 define the oxygen discharge chamber 13.

The first and second gas chambers 11 and 12 are defined by the first and second solid electrolyte layers 53 and 55 and the spacer 54. The reference gas chamber 14 is defined by the second solid electrolyte layer 55 and the heater 15 and leads to the air. The first gas chamber 11 leads to the second gas chamber 12 through a second diffusion resistance path 120 which is narrower than the first and second gas chambers 11 and 12.

The sensor cell 3 has, as described above, the negative electrode 31 exposed to the second gas chamber 12 and the positive electrode 32 exposed to the oxygen discharge chamber 13. Disposed between the negative electrode 31 and the positive electrode 32 is a senor circuit 35 consisting of a current detector 351 (i.e., ammeter) and a power supply 352. The negative electrode 31 is made of platinum which is active with respect to NOx.

The monitor cell 6 has the negative electrode 61 exposed to the second gas chamber 12 and the positive electrode 62 exposed to the oxygen discharge chamber 13. Disposed between the negative electrode 61 and the positive electrode 62 is a monitor circuit 65 consisting of a current detector 651 (i.e., ammeter) and a power supply 652. The negative electrode 61 is implemented by a Pt-Au electrode which is inactive with respect to NOx.

A feedback circuit 655 is disposed between the current detector 651 and a power supply 252 of a pump circuit 25, as will be described later.

The pump cell 2 has the negative electrode 21 exposed to the first gas chamber 11 and the positive electrode 22 exposed to the reference gas chamber 22. Disposed between the negative electrode 21 and the positive electrode 22 is the pump circuit 25 which consists of a current detector 251 (i.e., ammeter) and the power supply 252. The negative electrode 21 is implemented by a Pt-Au electrode which is inactive with respect to NOx.

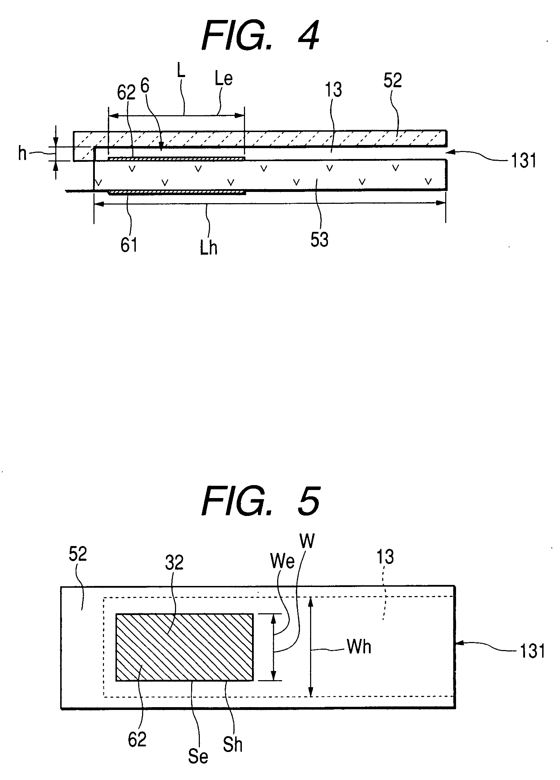

The oxygen discharge chamber 13 is, as clearly shown in Figs. 1 and 3 to 5, of a rectangular parallelopiped shape and leads outside the gas sensor 1 (i.e., the air) through an opening 131. The oxygen discharge chamber 13 has, as shown in Figs. 4 and 5, a height *h,* a length *Lh,* and a width *Wh* defined, as shown in Fig. 3, in a thicknesswise direction, a longitudinal direction, and a lateral direction of the gas sensor 1. The height his 0.15mm. The length *Lh* is 24mm. The width *Wh* is 2mm.

The oxygen discharge chamber 13 has a bottom area *Sh,* as defined by a broken line in Fig. 5, which is 48mm². The volume V of the oxygen discharge chamber 13 is, thus, given by *Sh* × *h =* 7.2mm3.

The positive terminal 32 of the sensor cell 3 and the positive terminal 62 of the monitor cell 6 are provided by a single rectangular plate. The rectangular plate may be covered partially with the spacer 52 to have at least a portion exposed inside the oxygen discharge chamber 13. Here, the exposed portion is defined by a length *Le,* as shown in Fig. 4, and a width *We,* as hatched in Fig. 5, and has an area *Se*. A total length, a total width, and a total area of the rectangular plate are defined as *L, W*, and *S*, respectively. In this embodiment, the whole of the rectangular plate is exposed inside the oxygen discharge chamber 13. Thus, the length *Le* is equal to the total length *L* which is 2.8mm. The width *We* is equal to the total width *W* which is 1.6mm. The total area *Se* is equal to the total area *S* which is 4.48mm².

The sensor cell 3 is responsive to application of a constant voltage from the power supply 352 to dissociate or ionize NOx through the negative electrode 31 to produce oxygen ions. The oxygen ions travel to the positive electrode 32 through the solid electrolyte layer 53 and become oxygen molecules on the positive electrode 32. The traveling of the oxygen ion produces an oxygen ion current which is measured by the current detector 351.

The negative electrode 31 of the sensor cell 3 also dissociates or ionizes oxygen molecules within the second gas chamber 12 in addition to NOx. The monitor cell 6 works to produce a current as a function the concentration of oxygen (O₂) within the second gas chamber 12 upon application of the voltage from the power supply 652. The output of the monitor cell 6 is measured by the feedback circuit 655 through the current detector 651. The feedback circuit 655 controls the voltage to be applied across the electrodes 21 and 22 of the pump cell 2 as a function of the output of the monitor cell 6 (i.e., the concentration of oxygen in the second gas chamber 12) to keep, as described above, the concentration of oxygen within the first gas chamber 11 constant. Therefore, the accurate measurement of the concentration of NOx within the second gas chamber 12 using the oxygen ion current produced by the negative electrode 31 of the sensor cell 3 is accomplished by finding the reading on the current detector 651 in advance in a condition that the concentration of NOx within the second gas chamber 12 is zero (0) and correcting the output of the sensor cell 3 so as to compensate for the reading or alternatively correcting and subtracting the output of the monitor cell 6, that is, the concentration of oxygen remaining within the second gas chamber 12 from the output of the sensor cell 3.

The feedback circuit 655, as described above, controls the power supply 252 so as to keep the output of the monitor cell 6 constant, thereby maintaining the concentration of oxygen within the second gas chamber 12 as well as the first gas chamber 11 at a constant level.

The positive electrodes 32 and 62 of the sensor cell 3 and the monitor cell 6 are, as described above, exposed inside the oxygen discharge chamber 13. The oxygen molecules appearing on the positive electrodes 32 and 62 are, thus, discharged outside the gas sensor 1 through the oxygen discharge chamber 13, thereby preventing the oxygen molecules from staying around the positive electrodes 32 and 62. This avoids oxygen enrichment and pressure elevation in an ambient atmosphere around the electrodes 32 and 62 which may produce an adverse effect on the outputs of the sensor cell 3 and the monitor cell 6.

The inventors of this application researched readings on the current detector 351 indicative of outputs of the sensor cell 3 for different values of the height *h* of the oxygen discharge chamber 13 and ratios *Le*/*L* and *We*/*W*.

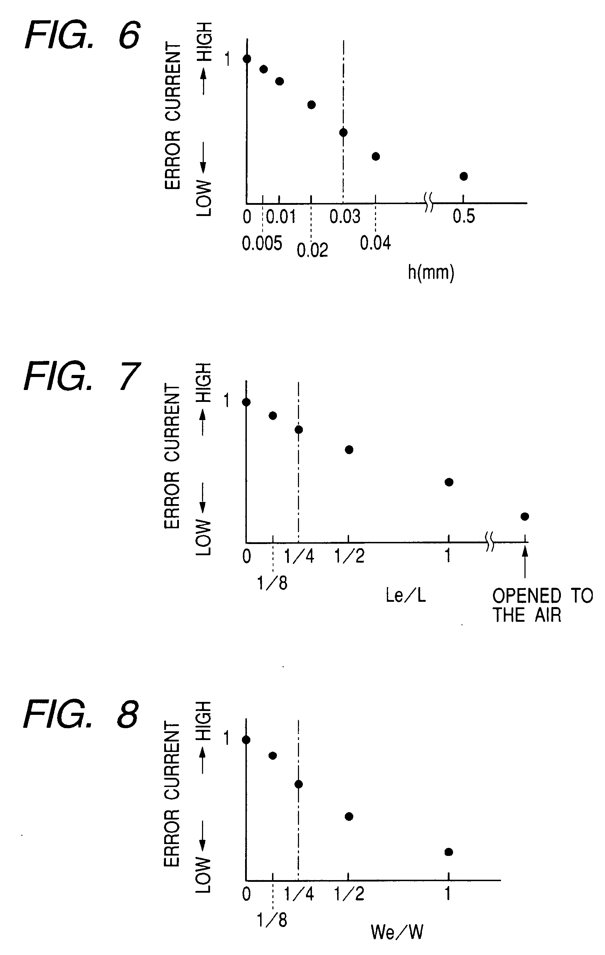

Figs. 6, 7, and 8 are graphs which indicate error currents outputted by the sensor cell 3 for different values of the height *h* and the ratios *Le*/*L* and *We*/*W,* respectively. Each of the error currents is given by a difference between an output of the current detector 351 which has measured the concentration of NOx in the second gas chamber 12 actually and an output of the current detector 351 estimated using an output of another means which has measured the concentration of NOx contained in the measurement gas before entering the second gas chamber 12. The numeral one (1) on an ordinate axis in each graph represents the error current in a case where the gas sensor 1 does not have the oxygen discharge chamber 13, that is, where the positive electrodes 32 and 62 of the sensor cell 3 and the monitor cell 6 are covered directly with the spacer 52 without any clearance therebetween.

The graphs of Figs. 6 to 8 show that the error currents are decreased by exposing as much area of the positive electrode 32 of the sensor cell 3 as possible to the oxygen discharge chamber 13, and particularly that the error currents decrease greatly when the height h of the oxygen discharge chamber 13 is 0.03mm or more, the ratio *Le*/*L* is 1/4 or more, and the ratio *We*/*W* is 1/4 or more, thereby resulting in greatly improved accuracy of measurement of NOx.

The rightmost plot on the graph of Fig. 7 indicates the error current in a case where the oxygen discharge chamber 13 leads to the air and the value of *Le*/*L* is one (1). It is, thus, appreciated that establishment of communication between the oxygen discharge chamber 13 and the outside of the gas sensor 1 serves to decrease the error current of the sensor cell 3 greatly.

We also performed measurement tests, as discussed below, and researched effect of the oxygen discharge chamber 13 on an output of the gas sensor 1 indicative of the concentration of NOx.

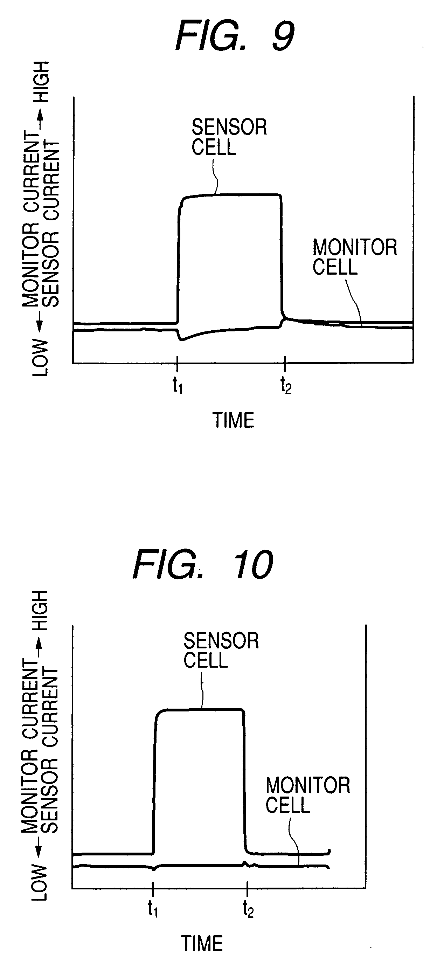

Fig. 9 indicates readings on the current detectors 351 and 651 when the measurement gas within the second gas chamber 12 is sensed using a gas sensor identical in structure with the gas sensor 1 except that the positive electrodes 32 and 62 of the sensor cell 3 and the monitor cell 6 are covered directly with the spacer 52 without formation of the oxygen discharge chamber 13. Fig. 10 indicates readings on the current detectors 351 and 651 using the gas sensor 1 which has the opening 131 of the oxygen discharge chamber 13 closed by a dense member.

We prepared a nitrogen gas, a NOx gas, and a mixture thereof as the measurement gas.

First, only the nitrogen gas was supplied to the gas sensor 1. When time *t1* was reached, the mixture of the nitrogen and NOx gasses was supplied to the gas sensor 1. The supply of only the NOx gas was stopped at time t2.

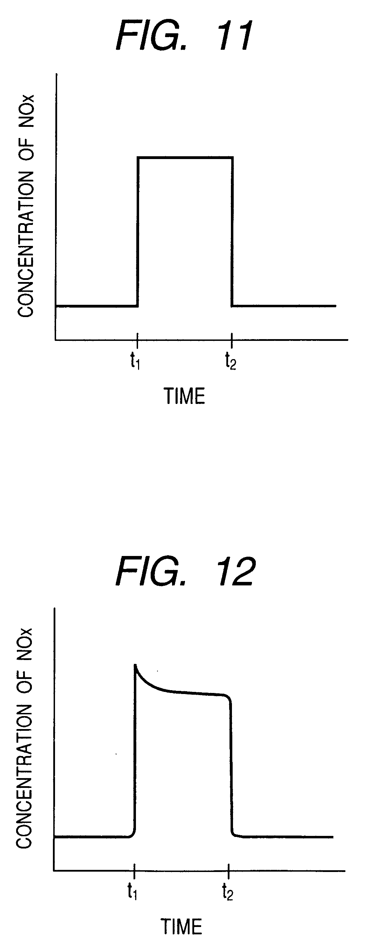

Fig. 11 illustrates the concentration of NOx determined by processing outputs of the current detectors 351 and 651, as shown in Fig. 10, using an NOx concentration determining circuit (not shown). Fig. 12 illustrates the concentration of NOx determined by processing outputs of the current detectors 351 and 651, as shown in Fig. 9, using the NOx concentration determining circuit.

The graph of Fig. 9 shows that the absence of the oxygen discharge chamber 13 causes the output of the monitor cell 6 (i.e., a monitor current) to drop immediately after the NOx gas enters the gas sensor 1 and return or increase gradually, but it rises suddenly at the instance the supply of the NOx gas is stopped. The drop in output of the monitor cell 6 arises from the fact that when the NOx gas enters the second gas chamber 12, oxygen molecules of NOx travel to the positive electrode 62, but the interior of the positive electrode 62 will be filled with the oxygen molecules immediately, thereby causing the oxygen molecules to return back to the negative electrode 61 as a minus current which is added to the output of the monitor cell 6. The presence of the oxygen discharge chamber 13, as apparent from the graph of Fig. 10, causes the output of the monitor cell 6 to experience a similar change, but the magnitude thereof is small. Comparison between the graphs of Figs. 9 and 10 also shows that the absence of the oxygen discharge chamber 13 results in a great variation in output of the sensor cell 3.

Comparison between the graphs of Figs. 11 and 12, therefore, shows that the absence of the oxygen discharge chamber 13 results in great variations in measured value of concentration of NOx when the NOx gas is supplied to the gas sensor 1 and when such supply is stopped, thereby causing an error of an output of the gas sensor 1 to increase.

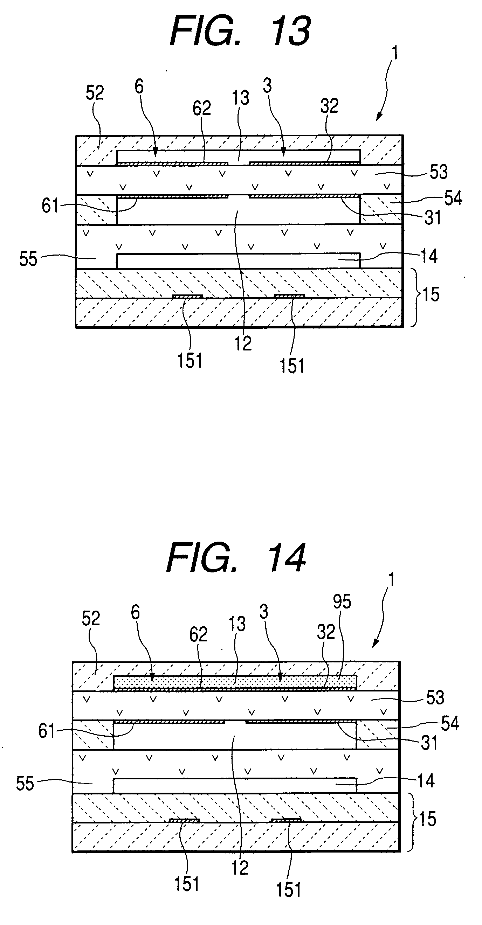

Figs. 13 and 14 show modifications of the sensor cell 3 and the monitor cell 6.

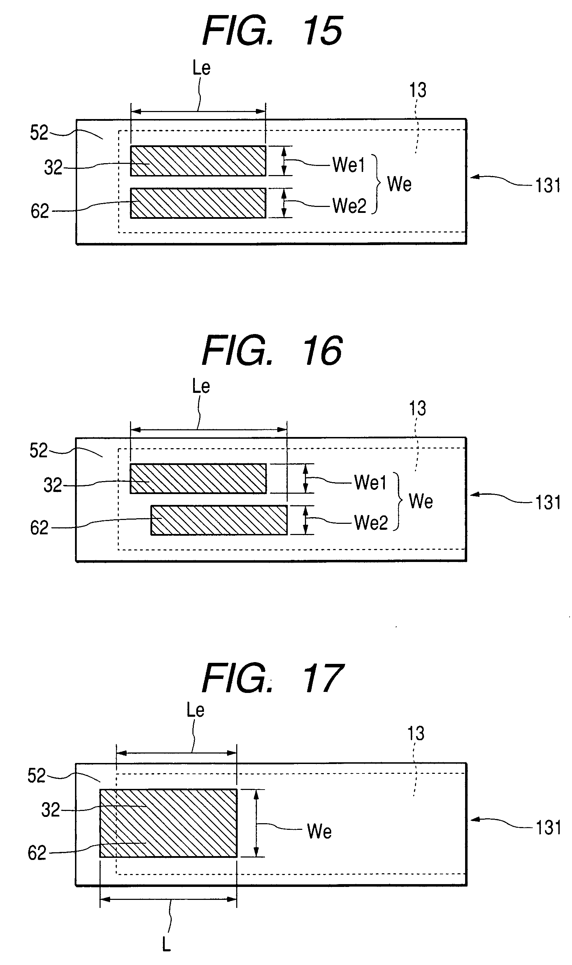

In Fig. 13, the positive electrodes 32 and 62 are provided by discrete electrodes. In this modification, the positive electrodes 32 and 62 may, as shown in Fig. 15, extend in parallel to each other at the same location in the longitudinal direction of the gas sensor 1. The length *Le,* as described above, is equivalent to the length of each of the positive electrodes 32 and 62.

The positive electrodes 32 and 62 may, as shown in Fig. 16, be arranged at different locations in the longitudinal direction of the gas sensor 1. In this case, the length *L* is defined by the distance between the left end, as viewed in the drawing, of the electrode 32 and the right end of the electrode 62.

In the modifications, as shown in Figs. 13, 15, and 16, the total width *We* is defined by the sum of widths *We1* and *We2* of the electrodes 32 and 62.

The positive electrodes 32 and 62 may alternatively be, as shown in Fig. 14, covered directly with a porous member 95 disposed within the interior of the gas discharge chamber 13 without any clearance.

The positive electrodes 32 and 62 may also, as shown in Fig. 13, extend partially outside the oxygen discharge chamber 13.

While the present invention has been disclosed in terms of the preferred embodiments in order to facilitate better understanding thereof, it should be appreciated that the invention can be embodied in various ways without departing from the principle of the invention. Therefore, the invention should be understood to include all possible embodiments and modifications to the shown embodiments which can be embodied without departing from the principle of the invention as set forth in the appended claims.

A gas sensor is provided which includes a sensor cell. The sensor cell is made up of a solid electrolyte member and a positive and a negative electrode installed on the solid electrolyte member. The sensor cell works to produce an electric current produced by a flow of oxygen ions from the negative to positive electrode which arises from dissociation of oxygen molecules on the negative electrode. An oxygen discharge chamber is provided on the positive electrode to discharge the oxygen molecules from outside the positive electrode, thereby eliminating an error of a sensor output caused by a stay of the oxygen molecules around the positive electrode.

## Claims

1. A gas sensor comprising:
a gas chamber into which a measurement gas is introduced from outside the gas sensor;
a sensor cell made up of a solid electrolyte member and a positive and a negative electrode installed on the solid electrolyte member, said sensor cell working to produce an output as a function of concentration of a specified component of the measurement gas;
a monitor cell made up of a solid electrolyte member and a positive and a negative electrode installed on the solid electrolyte member, said monitor cell working to produce an output as a function of concentration of oxygen within said gas chamber;
a pump cell made up of a solid electrolyte member and a pair of oxygen pumping electrodes installed on the solid electrolyte member, said pump cell working to pump oxygen out of and into said gas chamber, selectively; and
an oxygen discharge chamber to which a portion of the positive electrode of at least one of said sensor cell and said monitor cell is exposed, said oxygen discharge chamber being defined by a member which is so dense as to arrest penetration of oxygen molecules therethrough.

2. A gas sensor as set forth in claim 1, wherein the solid electrolyte members of said monitor cell and said sensor cell have outer surfaces which face the measurement gas existing outside the gas sensor and on which the positive electrodes of said monitor cell and said sensor cell are installed, and wherein the member is installed on the outer surfaces of the solid electrolyte members to define said oxygen discharge chamber to which oxygen molecules appearing on the portion of the positive electrode of the at least one of said sensor cell and said monitor cell are discharged.

3. A gas sensor as set forth in claim 1, wherein if a volume of said oxygen discharge chamber is defined as *V*, and an area of the portion of the positive electrode of the at least one of said sensor cell and said monitor cell exposed inside the oxygen discharge chamber is defined as *Se*, then a relation of 0.01 ≦ *V*/*Se* is met.

4. A gas sensor as set forth in claim 1, wherein a height of said oxygen discharge chamber is 0.01mm or more.

5. A gas sensor as set forth in claim 1, wherein if a total area of the positive electrode of the at least one of said sensor cell and said monitor cell is defined as *S*, and an area of the portion of the positive electrode of the at least one of said sensor cell and said monitor cell exposed inside the oxygen discharge chamber is defined as *Se,* then a relation of 0.25 ≦ *Se*/*S* is met.

6. A gas sensor as set forth in claim 1, wherein if a total length of the positive electrode of the at least one of said sensor cell and said monitor cell extending in a lengthwise direction of the gas sensor is defined as *L*, and a length of the portion of the positive electrode of the at least one of said sensor cell and said monitor cell exposed inside the oxygen discharge chamber extending in the lengthwise direction of the gas sensor is defined as *Le,* then a relation of 0.25 ≦ *Le*/*L* is met.

7. A gas sensor as set forth in claim 1, wherein if a total width of the positive electrode of the at least one of said sensor cell and said monitor cell extending perpendicular to a length of the gas sensor is defined as *W*, and a width of the portion of the positive electrode of the at least one of said sensor cell and said monitor cell exposed inside the oxygen discharge chamber extending perpendicular to the length of the gas sensor is defined as *We,* then a relation of 0.25 ≦ *We*/*W* is met.

8. A gas sensor comprising:
a sensor cell made up of a solid electrolyte member and a positive and a negative electrode installed on the solid electrolyte member, said sensor cell working to produce an output as a function of concentration of a specified component of a measurement gas;
a monitor cell made up of a solid electrolyte member and a positive and a negative electrode installed on the solid electrolyte member, said monitor cell working to produce an output as a function of concentration of oxygen contained in the measurement gas; and
an oxygen discharge chamber to which a portion of the positive electrode of at least one of said sensor cell and said monitor cell is exposed.

9. A gas sensor as set forth in claim 8, wherein the solid electrolyte members of said monitor cell and said sensor cell have outer surfaces which face the measurement gas existing outside the gas sensor and on which the positive electrodes of said monitor cell and said sensor cell are installed, and wherein said oxygen discharge chamber is defined by a member which is installed on the outer surfaces of the solid electrolyte members and works to discharge oxygen molecules appearing on the portion of the positive electrode of the at least one of said sensor cell and said monitor cell.

10. A gas sensor as set forth in claim 8, wherein if a volume of said oxygen discharge chamber is defined as *V*, and an area of the portion of the positive electrode of the at least one of said sensor cell and said monitor cell exposed inside the oxygen discharge chamber is defined as *Se*, then a relation of 0.01 ≦ *V*/*Se* is met.

11. A gas sensor as set forth in claim 8, wherein a height of said oxygen discharge chamber is 0.01mm or more.

12. A gas sensor as set forth in claim 8, wherein if a total area of the positive electrode of the at least one of said sensor cell and said monitor cell is defined as *S*, and an area of the portion of the positive electrode of the at least one of said sensor cell and said monitor cell exposed inside the oxygen discharge chamber is defined as *Se*, then a relation of 0.25 ≦ *Se*/*S* is met.

13. A gas sensor as set forth in claim 8, wherein if a total length of the positive electrode of the at least one of said sensor cell and said monitor cell extending in a lengthwise direction of the gas sensor is defined as *L*, and a length of the portion of the positive electrode of the at least one of said sensor cell and said monitor cell exposed inside the oxygen discharge chamber extending in the lengthwise direction of the gas sensor is defined as *Le,* then a relation of 0.25 ≦ *Le*/*L* is met.

14. A gas sensor as set forth in claim 8, wherein if a total width of the positive electrode of the at least one of said sensor cell and said monitor cell extending perpendicular to a length of the gas sensor is defined as *W*, and a width of the portion of the positive electrode of the at least one of said sensor cell and said monitor cell exposed inside the oxygen discharge chamber extending perpendicular to the length of the gas sensor is defined as *We,* then a relation of 0.25 ≦ *We*/*W* is met.
